# EUROPEAN PATENT APPLICATION

(11) **EP 4 799 658 A1**
(43) Date of publication of application: **02.09.2026**
(21) Application number: 25305260.9
(22) Date of filing: 27.02.2025
(51) Int. Cl.: A61L 27/26, A61L 27/52, A61L 27/56, B33Y 80/00

(54) **DRY IMPLANT**

(71) Applicant: Healshape, 69008 Lyon (FR)
(72) Inventor: CHERBLANC, Audrey, 69008 Lyon (FR); GODET, Baptiste, 69008 Lyon (FR); DURAND, Caroline, 69008 Lyon (FR); DOS SANTOS, Morgan, 69008 Lyon (FR); THÉPOT, Amélie, 69008 Lyon (FR); BRAC DE LA PERRIÈRE, Sophie, 69008 Lyon (FR)
(74) Representative: Icosa

(57) **Abstract**

The present disclosure relates to a dry implant suitable for rehydration before placement in a human or animal body.

## Description

### FIELD OF DISCLOSURE

The present disclosure relates to the field of medical technology, in particular to a three-dimensional medical implant for regeneration of soft tissue and intended to be introduced - or to allow study of such an introduction - into the human body or another living organism.

### BACKGROUND OF DISCLOSURE

The activity in the field of three-dimensional (3D) scaffolding structures for the purpose of bridging tissue gaps and giving the cells a scaffold to populate and proliferate on has been considerable, especially in the last 15 years. However, the limited number of such devices meeting the regulatory requirements in today's market indicates the difficulty in providing an implant that fits the surgeons' needs in terms of tissue regeneration, ease of use and problem-free healing. One of the greatest challenges for the ideal implant is that it shall combine an adequate modulus required by the surrounding tissue to avoid modulus mismatch; have biomimetic properties allowing cell adhesion and at the same time have an open structure to allow for tissue ingrowth and vascularization. Especially in soft tissue applications, the implant should be resilient enough to follow the placement in the living body and to regain shape with minimal hysteresis when no constraint is acting upon it until the implant has been fully populated by cells overtaking the load supporting function.

Hydrogels may seem to be the perfect choice of soft and resilient biomaterial, but they are fragile with poor mechanics and must usually be made and used at the bedside. In addition, hydrogel-based implant - especially when hydrogels are based on natural polymers - shall be sterilized. Most popular sterilization methods include radiation - for instance based on gamma rays - or plasma. Unfortunately, these methods usually lead to degradation of hydrogel mechanical properties. Besides, sterilization by gas diffusion process - for instance with ethylene-oxide - cannot be implemented in water-based implants, because reactive gas mainly reacts with water before reaching the microorganisms to be degraded.

There is thus a need for hydrogel-based implants retaining their mechanical properties after a classical sterilization step. In other words, the chemical structure of the implant should not be degraded in a way that decreases mechanical and/or biological properties.

It has been surprisingly found that a hydrogel based implant can be dried, sterilized then rehydrated to finally present suitable chemical and/or mechanical properties for implantation. Indeed, in the dry state, radiation used for sterilization does not interact with water, thus avoiding water radiolysis. Reactive gas like ethylene oxide may be used also, as interaction with water is avoided thus focusing ethylene oxide effect on polymers in the whole implant.

### SUMMARY

This disclosure thus relates to a dry implant comprising a dried hydrogel. The hydrogel comprises:
i) at least one biocompatible structuring agent, wherein said at least one biocompatible structuring agent is a polysaccharide network, and
ii) at least one biocompatible biological agent, wherein said at least one biocompatible biological agent is a native collagen network or a network of a derivative of collagen,

wherein said dry implant comprises at least one porous zone,
wherein the at least one porous zone comprises a plurality of pores, the at least one porous zone having a void fraction ranging from about 5% to about 99.5%.

In an embodiment, the implant has an isoperimetric ratio greater than or equal to 0.3.

In another embodiment, the implant has an aspect ratio greater than or equal to 3.

In an embodiment, the at least one biocompatible structuring agent is selected from the group consisting of alginate, hyaluronic acid, cellulose and its derivatives, chitosan and its derivatives, dextran, heparan sulfate, agarose, chondroitin and chondroitin sulfate, glycogen, starch, carrageenan, pectin, xanthan gum, pullulan, gellan, guar guar, gum arabic, and combinations thereof. Preferably, the at least one biocompatible structuring agent is selected from the group consisting of alginate, hyaluronic acid, cellulose and its derivatives, and combinations thereof. More preferably, the at least one biocompatible structuring agent is alginate.

In an embodiment, the at least one biocompatible biological agent is selected from the group consisting of native collagen, gelatin, or chemically modified collagens; preferably said at least one biocompatible biological agent is gelatin.

In an embodiment, the at least one biocompatible structuring agent is alginate and the at least one biocompatible biological agent is gelatin.

In an embodiment, the at least one porous zone occupies 90% or more of the volume of the dry implant.

In an embodiment, the dry implant comprises at least one non-porous zone, said at least non-porous zone being partially surrounding the porous zone.

In an embodiment, the dry implant is sterile.

This disclosure also relates to a process for manufacturing a dry implant comprising successively:
a) a step of preparing a hydrogel, wherein said hydrogel comprises i) at least one biocompatible structuring agent, wherein said at least one biocompatible structuring agent is a polysaccharide, and ii) at least one biocompatible biological agent, wherein said at least one biocompatible biological agent is a native collagen or a derivative thereof,
b) a step of three-dimensionally shaping the hydrogel so as to form a three-dimensional body implant comprising at least one porous zone, the at least one porous zone comprises a plurality of pores, and the at least one porous zone having a percentage of void ranging from about 5% to about 95%,
c) a step of stabilizing the hydrogel,
d) a step of drying, preferably freeze-drying, the stabilized hydrogel to obtain a dry implant.

In an embodiment, the process for manufacturing a dry implant further comprises a step e) of sterilizing the dry implant, preferably the sterilization is done by one or more of the following methods: sterilization by irradiation, sterilization by plasma, sterilization by gas such as ethylene oxide, or sterilization by supercritical carbon dioxide.

In an embodiment, the at least one biocompatible structuring agent from step a) is selected from the group consisting of alginate, hyaluronic acid, cellulose and its derivatives, chitosan and its derivatives, dextran, heparan sulfate, agarose, chondroitin and chondroitin sulfate, glycogen, starch, carrageenan, pectin, xanthan gum, pullulan, gellan, guar guar, gum arabic, and combinations thereof, preferably wherein the at least one biocompatible structuring agent is alginate.

In an embodiment, the at least one biocompatible biological agent from step a) is selected from the group consisting of native collagen, gelatin, chemically modified collagens; preferably said at least one biocompatible biological agent is gelatin.

In an embodiment, the at least one biocompatible structuring agent from step a) is alginate and the at least one biocompatible biological agent from step a) is gelatin and in step c), alginate is cross-linked with at least one divalent cation, preferably calcium, and gelatine is cross-linked with transglutaminase.

In an embodiment, the dry implant obtained in step d) or e) has an isoperimetric ratio greater than or equal to 0.3.

In another embodiment, the dry implant obtained in step d) or e) has an aspect ratio greater than or equal to 3.

This disclosure finally relates to a three-dimensional implant obtainable by a process for manufacturing disclosed hereabove.

### DEFINITIONS

In the present invention, the following terms have the following meanings:

"Absorbable" means the ability to be destroyed by a living organism. In particular when the implant is implanted in the host, said implant is absorbable if it is capable of being absorbed by said host.

"Aspect ratio" in relationship with an elongated three-dimensional implant refers to the ratio of the length of the implant along the longitudinal axis to the mean perpendicular diameter - or equivalent diameter for a non-circular section - of the implant. For instance for an ellipsoid having a main axis of 10 cm and the other axis of 1 cm and 3 cm, the aspect ratio is 10/[½(1+3)]=5. The aspect ratio is especially suitable to characterize implants such as veins/arteries, tendons, ligaments, nervous fibers...

**"Cross-linking agent",** in the context of the present disclosure, means an agent capable of cross-linking hydrogel components.

**"Dry"** in reference to a hydrogel, means that the water content is less than 15% in weight of the hydrogel.

**"Filling rate"** refers to the ratio of the volume of hydrogel - be it dry or hydrated - to the volume of the implant. The filling rate and the void fraction sum up to 100%.

**"Implant"** refers to a structure intended to represent in volume a part of an animal or human body. An implant may be intended to be implanted. Alternatively, an implant may be used to study *in vitro* the mechanisms expected if said implant was really implanted.

**"Implant surface"** refers to the surface of the envelope that surrounds the implant.

**"Implant volume"** refers to the volume comprised inside the envelope that surrounds the implant.

**"Isoperimetric ratio":** in relationship with a three-dimensional implant refers to the ratio ${q}_{1} = \frac{36 {πV}^{2}}{{S}^{3}}$ where S and V are respectively the surface and the volume of the envelope surrounding the three-dimensional implant. Indeed, the internal porosity of the implant shall not be considered here, but only the external shape. The isoperimetric ratio is especially suitable to characterize implants such as breast implants, gluteal implants, implants designed to replace a tumor or loss of substance.

**"Overall pore size",** in the context of the present disclosure, refers to the average of the pore size values measured over the porous zone of the implant. It does not refer to the size of pores of the hydrogel itself - even in the dry state.

**"Overall porosity",** in the context of the present disclosure, refers to the porosity at a macroscopic scale, linked to the structure of the aerated material of the porous zone of the implant. It does not refer to the porosity of the hydrogel itself - even in the dry state. When the adjective "porous" is used, it only refers to the overall porosity.

**"Pore size":** in the context of the present disclosure, refers to the largest distance between two opposing hydrogel filaments in the aerated structure of the porous zone.

**"Shaping",** in the context of the present disclosure, consists in giving the hydrogel a particular shape and structure or architecture, and in particular adapted to the destination of the hydrogel once dried and further rehydrated.

**"Void fraction"** refers to the ratio of the porous volume of the implant - be it dry or hydrated - to the volume of the implant. The void fraction and the filling rate sum up to 100%.

### DETAILED DESCRIPTION

The present invention makes it possible to provide dry implants, which may be sterilized in the dry form, then rehydrated to restore the implants shape and mechanical characteristics, especially in terms of the mechanical strength of the constituents, which is similar to that of native tissues they intend to replace, especially stability over time - during storage, implantation and until the implant resorbs in the body of the patient - and resistance to tearing.

The implants herein disclosed can be used, after rehydration, in place of (replacement of all or part of) or in addition to (augmentation of) various organs or tissues of the animal body and more particularly of the human body, either permanently or temporarily.

The implant can also be used *in vitro,* to study the mechanisms that are expected *in vivo.* For instance, the implant may be used as a scaffold for cell culture and evaluation of integration of an implant in the animal or human body by determination of colonization, shape change... For instance, the implant may be used to mimic a tumor and study the reaction of said tumor to a treatment, chemical or surgical.

By definition the rehydrated implants according to the invention are suitable for contact with living fluids or living tissues. In particular, they are intended to be implanted under the skin, or even on the skin, especially for skin regeneration and/or healing.

The rehydrated implants herein disclosed are therefore substitutes or additions, replacing and/or increasing and/or reinforcing soft or flexible, and sometimes elastic, tissues. They are preferably used to replace entirely or partially, to increase or to reinforce connective tissues, skin and adipose tissues.

The rehydrated implants herein disclosed are therefore intended for plastic, reconstructive or regenerative surgery.

All the constituents of the implants described below must meet the regulatory requirements specific to devices intended to be implanted, particularly with respect to purity grades.

### Dry implant

This disclosure relates to a dry implant. The dry implant can be sterilized with usual techniques, without altering the chemical and/or mechanical properties of the implant obtained after rehydration.

Further, the dry hydrogel comprises
i. at least one biocompatible structuring agent, wherein said at least one biocompatible structuring agent is a polysaccharide network
ii. at least one biocompatible biological agent, wherein said at least one biocompatible biological agent is a native collagen network or a network of a derivative of collagen.

In addition, the dry implant comprises at least one porous zone. Said porous zone comprises a plurality of pores and has a void fraction ranging from 5% to 99.5%. In other words, a part of the dry implant is an aerated material made of a scaffold defining open cells, *i.e.,* cells in which the walls are partially opened like a sponge. The scaffold is typically made of hydrogel filaments.

The dry hydrogel is obtained by removal of water from a hydrogel by drying for instance by vacuum drying, or by freeze drying, also known as lyophilization. In this disclosure, a hydrogel is said "dry" when the water content is less than 15% in weight of the hydrogel.

### Dry implant shape

During drying process, the implant undergoes mechanical constraints. Indeed, water removal induces an overall contraction, typically in a range from 3% to 10% in each direction, leading to a volume contraction in a range from 10% to 25%. In such circumstances, an aerated material is prone to deform and optionally break. Three different situations may be considered. Hereafter, the shape of the implant will be defined by the surface and volume of the envelope that surrounds the implant: notwithstanding its porous nature, the voids of the porous zone are considered here as being part of the implant.

Some implants have a regular shape. Here, regular means that the characteristic lengths of the implant in the three directions are of the same order of magnitude. In such implants, stress/strain generated during drying cannot be relaxed in a specific direction, and breakage occurs often in the most stressed filaments. Therefore, after rehydration, the mechanical properties of the implant are degraded. In this disclosure, such regular shape is defined with the isoperimetric ratio. In this disclosure, the regular shape corresponds to breast implants, pectoral implant, calf implant, abdominal implant, gluteal implant, cheekbone, soft tissue defects - for instance encountered in the setting of trauma, infection, burns or resection of tumors -, loss of substance - due to disease, injury, trauma or congenital anomalies for instance. Implants with a regular shape and the specific composition disclosed hereabove withstand stress/strain generated during drying without breakage, and lead to implants with suitable mechanical properties after rehydration. In an embodiment, the implant has an isoperimetric ratio greater than or equal to 0.3.

Some implants have a laminar shape. Here, laminar means that one characteristic length of the implant is much less - along the short axis - than the two other characteristic lengths - the main plane. The laminar shape corresponds to sheets or objects that are formed by bending a sheet. In such implants, stress/strain generated during drying is preferentially relaxed in the short axis direction, *i.e.,* orthogonally to the main plane. Usually, implant is very slightly deformed - bent or twist actually - and no breakage occur. After rehydration, mechanical properties are usually maintained. In this disclosure, the laminar shape corresponds to implants encountered in the setting of severe burns for instance.

Some implants have an elongated shape. Here, elongated means that one characteristic length of the implant is much greater - along the longitudinal axis - than the two other characteristic lengths - defining the cross section. In such implants, stress/strain generated during drying generally leads to twirling or high bend, and optionally breakage. After rehydration, the shape is usually not recovered, and the implant cannot be used. In this disclosure, an elongated shape is defined with the aspect ratio. In this disclosure, the elongated shape corresponds to nerves, venous, arteries, tendons, ligaments, tracheal or bronchial implants. Implants with an elongated shape and the specific composition disclosed hereabove withstand stress/strain generated during drying without breakage nor irreversible deformation, and lead to implants with suitable mechanical properties after rehydration. In an embodiment, the implant has an aspect ratio greater than or equal to 3, preferably greater than or equal to 5, more preferably greater than or equal to 8.

### Biocompatible structuring agent

In this disclosure, the dry hydrogel comprises at least one biocompatible structuring agent, selected from the family of polysaccharide network. Suitable polysaccharide may be selected from the group consisting of alginate, hyaluronic acid, cellulose and its derivatives, chitosan and its derivatives, dextran, heparan sulfate, agarose, chondroitin and chondroitin sulfate, glycogen, starch, carrageenan, pectin, xanthan gum, pullulan, gellan, guar guar, gum arabic, and combinations thereof. Polysaccharides are especially suitable due to their biocompatibility, low toxicity, relatively low cost and easy sourcing, high water uptake, gelation potential, viscosity and flexibility, and resistance. The biocompatible structuring agent provides a scaffold and the mechanical properties expected for the implant after rehydration.

In an embodiment, polysaccharide is selected from the group consisting of alginate, hyaluronic acid, cellulose and its derivatives, chitosan and its derivatives, dextran, heparan sulfate, and combinations thereof. These specific polysaccharides have demonstrated improved tissue regeneration.

Most suitable polysaccharides are selected from the group consisting of alginate, hyaluronic acid, cellulose and its derivatives, and combinations thereof. These specific polysaccharides have demonstrated a large improvement in tissue regeneration. The most suitable polysaccharide is alginate.

The biocompatible structuring agent is present in the hydrogel in the form of a network. This means that polysaccharide chains are bound one with another to form nodes, the segments of polysaccharide chains between nodes being edges. When polysaccharide chains are structured in fibers, the network is defined by bounds between fibers - nodes - and fiber edges between nodes. Hereafter, chains and fibers are used in an equivalent manner. The chemical nature of the node may be of various types. Nodes may be intermolecular or intramolecular. Nodes may result from entanglement of polysaccharide chains. Nodes may be covalent bounds, obtained directly between or inside the polysaccharide chains or obtained through a cross-linker agent, said crosslinker being covalently bound to two polysaccharide chains or two parts of a single polysaccharide chain. Nodes may result from various interactions, either driven by electric charges - ionic bounds, electrostatic bounds on complexation with charged centers - directly between the polysaccharide chains or obtained through an ionic compound - especially a cationic compound - or driven by polarization interactions.

In an embodiment, the biocompatible structuring agent is at least partially absorbable.

### Biocompatible biological agent

In this disclosure, the biocompatible biological agent is selected from the group consisting of collagen or derivatives thereof.

Collagen may be native, *i.e.,* isolated from natural source - animal or human. The native collagen may be further denatured or chemically modified.

Alternatively, collagen may be chemically synthesized, which includes without limitation peptide synthesis, recombinant collagen or cell-produced collagen. Such collagen may be further denatured or chemically modified.

Suitable collagen derivatives include gelatin and chemically modified collagen. Various chemical modification of collagen may be done to improve properties of the hydrated implant, especially to improve the growth of cells in the implant after introduction in the human or animal body.

Collagen may be partially hydrolyzed.

Collagen and derivatives may be modified by introduction of cross-linkers, to improve mechanical properties and limit - but not prevent totally - naturally occurring proteolysis, thus increasing the durability of the implant inside the human or animal body, even if the implant finally resorbs at least partially.

Collagen and derivatives may be modified by addition of functional groups, in particular functional groups selected from the group consisting of (meth)acrylates - such as methacrylated gelatin known under GelMa -, carbonyls, esters, acyls, amides or glycosides - for instance obtained by glycosylation. Such modification may change the initial properties of the hydrogel - before drying - especially hydrophily, solubility in water or water-based solvents, chemical interactions, resistance to degradation - such as oxidation or enzymatic breakage - or stability of the hydrogel in various conditions - temperature, ionic strength... Last, the modification may bring polymerizable functions to collagen and derivatives, allowing to form a network.

Collagen and derivatives may be modified by actinic irradiation, thus promoting either crosslinking of the collagen molecules or breakage of the collagen molecules to change mechanical properties or prevent degradation - by elimination of sites where natural degradation would otherwise occur.

In an embodiment, the biocompatible biological agent is gelatin.

Last, the biocompatible biological agent is present in the hydrogel in the form of a network. This means that collagen molecules - including all derivatives disclosed hereabove - are bound together to form nodes, the segments of collagen molecules between nodes being edges. Nodes may be intermolecular or intramolecular. The chemical nature of the node may be of various types. Nodes may result from entanglement of collagen molecules. Nodes may be covalent bounds, obtained directly between the collagen molecules - optionally via an enzyme creating a covalent bond - or obtained through a cross-linker agent, said crosslinker being covalently bound to two collagen molecules or two parts of a single collagen molecule. Nodes may result from various interactions, either driven by electric charges - ionic bounds, electrostatic bounds on complexation with charged centers - directly between the collagen molecules or obtained through an ionic compound or driven by polarization interactions.

**In** an embodiment, the biocompatible biological agent is at least partially absorbable.

### Suitable associations

**In** this disclosure, the weight concentration of the biocompatible structuring agent is ranging from 0,05% to 10%, preferably from 0.1% to 7.5%, or even more preferably from 0,1% to 5%, said weight concentration being defined as the weight of the biocompatible structuring agent divided by the weight of the hydrogel, *i.e.,* the sum of the weight of water - including salts - and the weight of the biocompatible structuring agent and the weight of the biocompatible biological agent.

**In** this disclosure, the weight concentration of the biocompatible biological agent is ranging from 0,1% to 25%, preferably from 0.5% to 20%, or even more preferably from 1% to 10%, said weight concentration being defined as the weight of the biocompatible structuring agent divided by the weight of the hydrogel, *i.e.,* the sum of the weight of water - including salts - and the weight of the biocompatible structuring agent and the weight of the biocompatible biological agent.

**In** this disclosure, the weight ratio of biocompatible structuring agent and biocompatible biological agent is preferably ranging from 1:0.2 to 1:50, more preferably ranging from 1:1 to 1:10. Said weight ratio is defined by the relative weight of both agents. For the sake of clarity, the composition A/B with a weight ratio of 1:2 means that the weight of agent A is half the weight of agent B in the hydrogel. For instance 1wt%A/2wt%B and 3wt%A/6wt%B both correspond to a composition A/B with a weight ratio of 1:2.

The following associations of biocompatible structuring agent and biocompatible biological agent are especially suitable, and yield efficient dry implants:
- Biocompatible structuring agent: alginate with biocompatible biological agent: gelatin, preferably with a weight ratio of 1:2.5 in the hydrogel. Typically, compositions of 1wt%alginate/2.5wt%gelatin or 1.5wt%alginate/3.75wt%gelatin or 2wt%alginate/5wt%gelatin are suitable.
- Biocompatible structuring agent: alginate with biocompatible biological agent: gelatin, preferably with a weight ratio of 1:3.33 in the hydrogel. Typically, compositions of 1wt%alginate/3.33wt%gelatin or 1.5wt%alginate/5wt%gelatin or 2wt%alginate/6.67wt%gelatin are suitable.
- Biocompatible structuring agent: alginate with biocompatible biological agent: gelatin, preferably with a weight ratio of 1:5 in the hydrogel. Typically, compositions of 1wt%alginate/5wt%gelatin or 1.5wt%alginate/7.5wt%gelatin or 2wt%alginate/10wt%gelatin are suitable.
- Biocompatible structuring agent: alginate with biocompatible biological agent: collagen, preferably with a weight ratio of 1:2.5 in the hydrogel. Typically, compositions of 1 wt%alginate/2.5wt%collagen or 1.5wt%alginate/3.75wt%collagen or 2wt%alginate/5wt%collagen are suitable.
- Biocompatible structuring agent: hyaluronic acid with biocompatible biological agent: gelatin, preferably with a weight ratio of 1:2.5 in the hydrogel. Typically, compositions of 1wt%hyaluronic acid/2.5wt%gelatin or 1.5wt% hyaluronic acid/3.75wt%gelatin or 2wt%hyaluronic acid/5wt%gelatin are suitable.
- Biocompatible structuring agent: cellulose or derivatives with biocompatible biological agent: gelatin, preferably with a weight ratio of 1:2.5 in the hydrogel. Typically, compositions of 1wt% cellulose/2.5wt%gelatin or 1.5wt%cellulose/3.75wt%gelatin or 2wt%cellulose/5wt%gelatin are suitable.
- Biocompatible structuring agent: chitosan or derivatives with biocompatible biological agent: gelatin, preferably with a weight ratio of 1:2.5 in the hydrogel. Typically, compositions of 1wt% chitosan/2.5wt%gelatin or 1.5wt%chitosan/3.75wt%gelatin or 2wt% chitosan/5wt%gelatin are suitable.

**In** an embodiment, the polysaccharide network and the collagen network are not independent, *i.e.,* they overlap - including entanglement - in the hydrogel without any specific interactions.

Alternatively, the polysaccharide network and the collagen network have specific interaction, *i.e.,* they form mixed nodes where a polysaccharide chain and a collagen molecule are bound. Mixed nodes may be covalent bounds, obtained directly between the collagen molecule and the polysaccharide chain or obtained through a cross-linker agent, said crosslinker being covalently bound to a collagen molecule and a polysaccharide chain. Mixed nodes may be intermolecular interactions, either driven by electric charges - ionic bounds, electrostatic bounds on complexation with charged centers - directly between a collagen molecule and a polysaccharide chain or obtained through an ionic compound - especially a cationic compound - or driven by polarization interactions.

### Additives

Additives may be added to the hydrogel to impart specific properties, especially to reinforce the mechanical properties and/or biological properties. These additives may be contributing to the hydrogel structure or reinforcing the hydrogel, for instance polymers or textile fibers.

Additives contributing to the hydrogel structure may be components extracted from extracellular matrix such as laminin, entactin, elastin, vimentin, keratin, fibronectin, vitronectin, glycosaminoglycans including heparin, or proteoglycans such as Matrigel. Other suitable additives are plasma proteins such as albumin, fibrinogen and its derivatives - fibrin for instance. Other suitable additives are fibroin, self- assembling peptides and peptide units such as short di-peptides (FF), Fmoc-peptide-based hydrogels such as Fmoc-FF-OH, Fmoc-FRGD-OH, Fmoc-RGDF-OH, Fmoc-2-Nal-OH, Fmoc-FG-OH, PuraMatrix, MAX1, KFE8, EAK16-II, PLL (Poly-L-Lysine), IKVAV, RADA-16-1, RGD. Other suitable additives are polyethyleneglycol (PEG) and their derivatives, polyvinylalcohol (PVA), polyvinylpyrrolidone (PVP), or polyurethane (PU). Other suitable additives are polyacrylamide (PAAm), poly-N-Isopropylacrylamide) (PNIPAM), polyacrylic acid (PAA), polyhydroxyethylmethacrylate (PHEMA), polyoligoethyleneglycol Methacrylate, poly-2-hydroxyethyl acrylate (PHEA), polymethacrylic acid (PMAA), polymethylmethacrylate (PMMA).

The additives listed hereabove as contributors to the hydrogel structure may be further chemically modified with the addition of a methacrylate group.

Said additives contributing to the hydrogel structure may be present with a weight concentration ranging from 0,001% to 50%, preferably from 0.01% to 25%, or even more preferably from 0,1% to 10%, said weight concentration being defined as the weight of the additive divided by the sum of the weight of the biocompatible structuring agent and the weight of the biocompatible biological agent.

Suitable polymers are polyolefin such as polyethylene (PE), polypropylene (PP), polytetrafluoroethylene (PTFE), polyvinylchloride (PVC) ; polyesters such as polyethylene terephthalate (PET - including polymers sold under the Dacron brand name), polybutylene terephthalate (PBT), polyglycolic acid (PGA), polylactic acid and derivatives (PLA), poly-L-lactic acid (PLLA), poly-D-lactic acid (PDLA), copolymer of L-lactic acid and D-lactic acid (PDLLA), copolymer of lactic acid and glycolic acid (PLGA), polydioxanone (PDO), polycaprolactone and derivatives (PCL), copolymers of glycolide and caprolactone (PGACL), poly-4-hydroxybutyrate (P4HB); polyethers such as polyether ether ketone (PEEK), poly ether sulfones (PES), poly ethylene oxide (PEO), poly propylene oxide (PPO); polyester amides; polyamides; polyurethanes, polycarbonates, polyethylene glycol diacrylate (PEGDA); poly glycerol sebacate (PGS); block copolymers of ethylene oxide and propylene oxide such as copolymers sold under the Pluronic brand name, especially Pluronic F127; copolymer of 1,8-octanediol and citric acid; poly diol citrate; or mixtures of at least two of these polymers. Said polymers may be present in weight concentrations ranging from 0,001% to 50%, preferably from 0.01% to 25%, or even more preferably from 0,1% to 10%, said weight concentration being defined as the weight of the additive divided by the sum of the weight of the biocompatible structuring agent and the weight of the biocompatible biological agent.

Suitable textile fibers are of natural origin such as cellulose fibers; of synthetic origin such as polyester fibers, polyamide fibers - for instance known as nylon fibers -, polyethylene fibers, polypropylene fibers, and acrylic fibers. Said textile fibers may be present at a weight concentration of less than 20%, preferably less than 10%, most preferably less than 5%, said weight concentration being defined as the weight of the additive divided by the sum of the weight of the biocompatible structuring agent and the weight of the biocompatible biological agent. According to one embodiment, the dry implants of the invention do not comprise textile fibers, whether of natural or synthetic origin.

### Porous zone

The dry implant of the disclosure is porous. Two scales of porosity have to be clearly distinguished.

The dry implant is an aerated structure and thus comprises open pores, *i.e.,* pores in which the walls are partially opened. In other words, there are continuous paths throughout the dry implant - or a part of the dry implant - between neighboring pores. Indeed, the implant may comprise porous parts separated by non-porous parts. The cross sections of said paths are in a micrometric to millimetric scale, mainly controlled by the manufacturing process of the implant. For instance, with 3D printing, pore size is defined by the distance between the neighboring filaments of material. This porosity is named overall porosity in this disclosure.

In other words, the overall porosity corresponds to the void fraction of the implant and is not affected by the drying/rehydration process, except for the contraction associated to drying. In particular, the overall porosity after rehydration should be comparable to the porosity of the implant before drying, as the overall porosity is a key feature of the implant. Indeed, the overall porosity defines some mechanical features, and defines the space that can be colonized by cells after placement of the implant in the human or animal body.

Besides, the open cells are delimited by hydrogel filaments comprising the biocompatible structuring agent, the biocompatible biological agent and a small amount of water - a filament is a macroscopic object comprising microscopic fibers and water. These hydrogel filaments are also porous, said porosity being generated during drying by evaporation of water leaving free space between the polysaccharide/collagen matrix. This porosity intrinsic to the hydrogel, is defined at a nanometric scale and is not considered in this disclosure.

For the sake of clarity, the hydrogel filaments in the dry state do define the geometry of the open cells of the dry implant and the overall porosity. But the hydrogel filaments in the dry state do not contribute to the overall porosity. When the adjective "porous" is used, it relates only to the overall porosity.

In this disclosure, the dry implant comprises one porous zone. If a plurality of separated porous zones is present, they are collectively referred to as the porous zone. In other words, a part of the dry implant is an aerated material comprising open cells. In an embodiment, the porous zone occupies 90% or more of the volume of the dry implant.

In particular, the dry implant may be totally porous, with a porous zone occupying 100% of the volume of the dry implant.

In an embodiment, the fraction of volume of the dry implant occupied by the porous zone may be 91%, 92%, 93%, 94%, 95%, 96%, 97%, 98% or 99%.

In one embodiment, for soft tissue for instance, a large overall pore size in the porous zone of the implant, in the range of 1000 µm to 10000 µm, in particular 1000 µm to 5000 µm, will be preferred, as the resulting implant, containing less material, will be more flexible. Preferably, the porous zone of the implant will have a fill rate of 5% to 50%, and even more preferably, 15% to 50%, where the fill rate is the ratio defined by the volume of hydrogel divided by the volume of the implant.

In one embodiment, for rigid tissue for instance, a low overall pore size in the porous zone of the implant, in particular less than 1000 µm, will be preferred as it will provide said implant with a high mechanical strength for rigid tissue. Preferably, the porous zone of the implant will have a fill rate of 50% to 99%, and even more preferably, 50% to 90%.

In one embodiment, the porous zone has an overall porosity of at most 10000 µm. According to an embodiment, the porous zone has an overall porosity of at most 5000 µm.

The porous zone may have an overall porosity of at least 10 µm, for example at least 50 µm. According to an embodiment, the porous zone has an overall porosity of at least 100 µm, and even more preferably at least 500 µm.

Thus, according to one embodiment, the overall porosity of the porous zone of the implants according to the invention can range from 10 µm to 1000 µm, or from 20 µm to 1000 µm, or from 1000 µm to 5000 µm.

Thus, according to one embodiment, the overall porosity of the porous zone of the implants according to the invention may range from 100 µm to 10000 µm, preferably from 500 µm to 250 µm, more preferably from 500 µm to 1000 µm or from 1000 µm to 2500 µm or from 2500 µm to 5000 µm.

### Non porous zone

When the dry implant is not totally porous, the dry implant comprises a non-porous zone. If a plurality of separated non-porous zones are present, they are collectively referred to as the non-porous zone. Typically, the non-porous zone is a thin but dense part. Such non-porous zone is useful to reinforce the structure of the dry implant, to ease handling of the dry implant or the implant after rehydration, or to avoid friction at the implant/tissue interface.

The repartition of the non-porous zone and the porous zone may have any structure.

Suitable structures for the non-porous zone are the following:
- The non-porous zone surrounds at least partially the porous zone;
- The non-porous zone splits the porous zone in a plurality of well separated zones;
- The non-porous zone mimics Cooper's ligaments from the breast anatomy to reinforce a breast implant mechanical resistance;
- The non-porous zone forms tubular structures, which can be used as injection channels for various purposes such as lipofilling.

### Sterilization

As already mentioned, the dry implant herein disclosed may be sterilized without degradation of its mechanical properties.

In an embodiment, the dry implant is sterile, as defined in the Sterility Assurance Level (SAL) standard.

The method of sterilization can be selected among the usual sterilization techniques suitable for medical devices. In particular, the following methods are suitable:
- Sterilization by irradiation, especially actinic radiation, gamma rays, X-ray, electron beam, or intense pulsed light.
- Sterilization by plasma, including cold plasma,
- Sterilization by gas, for instance with ethylene oxide
- Sterilization by supercritical carbon dioxide.

### Process for manufacturing a body implant

The disclosure also relates to a process for manufacturing a dry implant. The process comprises the following steps:

Hydrogel is prepared. The hydrogel comprises at least one biocompatible structuring agent, said at least one biocompatible structuring agent being a polysaccharide; and at least one biocompatible biological agent, said at least one biocompatible biological agent being a native collagen or a derivative thereof. All biocompatible structuring agents and biocompatible biological agents disclosed hereabove are suitable for the preparation of hydrogel.

Hydrogel is three-dimensionally shaped, leading to a three-dimensional body implant. Said implant comprises at least one porous zone, the at least one porous zone comprising a plurality of pores, and the at least one porous zone having a percentage of void ranging from about 5% to about 95%. Shaping is achieved by a 3D-printer suitable to print the hydrogel, with its specific rheological properties.

Hydrogel is then stabilized. By stabilization, it is meant that the biocompatible structuring agent is structured into a network, and the biocompatible biological agent is structured into a network. Both networks may be entangled or linked. All networking embodiments disclosed hereabove may be used in the stabilization step.

Stabilized hydrogel is then dried, preferably freeze-dried, leading to a dry implant.

The drying step is a low temperature drying process allowing to remove water by sublimation. The process may be run in two steps.

In a first step, the sample is frozen: temperature is decreased slowly from room temperature to a temperature in a range from -20°C to -60°C, the temperature decreases - hereafter a ramp - during 0 minute to 10 hours.. The freezing plateau following the freezing ramp may last from 10 minutes to 1 month. The freezing step may be uniform or adapted with several ramps and plateaus. In the frozen state, the implant may be stored for a long time, before drying *per se.*

Then, in a second step of primary drying, the frozen water - referred to as free water - is withdrawn by sublimation in a low temperature and low-pressure environment. Temperature range for primary drying is from -55°C to +40°C. Pressure range for primary drying step is from 5 µbar to 800 µbar. The primary drying plateau may last from 1 hour to 7 days. The primary drying step may be uniform or adapted with several temperature increase ramps and plateaus.

Optionally, a third step of additional drying allows withdrawal of water physically or chemically linked to the sample, in other words, desorption of water. This additional drying is run at intermediate temperature and low pressure. Temperature range for additional drying is from 0°C to +40°C. Pressure range for additional drying step is from 5 µbar to 800 µbar. The additional drying plateau may last from 1 hour to 7 days. Additional drying step may be uniform or adapted with several temperature increase ramps and plateaus.

For instance, implants are laid in a freeze-dryer and the following cycles are implemented:
- Freezing ramp from room temperature to -45°C in 225 min and then a plateau during 60 min.
- Primary drying step with several temperature increase ramps and plateaus from -45°C to +10°C in 18 hours, at 150 µbar and 55 hours of plateau at a temperature between -45°C and +10°C and 150 µbar.
- Additional drying step ramp from 10°C to 20°C in 4h, at 150 µbar, then a plateau for 20 hours at +20°C and 150 µbar.

Another protocol is the following:
- Freezing ramp from room temperature to -35°C in 60 min and then a plateau during 60 min.
- Primary drying step ramp from -35°C to -25°C in 3 hours, at 150 µbar, then a plateau for 55 hours at -25°C and 150 µbar.
- Additional drying step ramp from -25°C to 20°C in 20h, at 150 µbar, then a plateau for 20 hours at +20°C and 150 µbar.

After drying, the samples are stored at room temperature or +4/+8°C and at atmospheric pressure. The product may be freeze-dried directly on the shelves of a freeze-dryer, or may be placed in packaging with at least one moisture-permeable part, *e.g.,* Tyvek-type materials or PTFE materials.

### Use of dry implant

The dry implant hereabove disclosed may be used in various biological domains. Obviously, such dry implant may be intended for placement - after rehydration - in the body of a patient during a surgical operation, either for replacement of a tumor or a loss of substance, or for augmentation of various organs or tissues; either permanently or temporarily.

Alternatively, dry implants may be also used as models for *in vitro* studies, to explore the mechanisms that are expected *in vivo.* For instance, these implants may be models to study the biological process of tumor growth and angiogenesis. For instance, these implants may be models of an existing tumor for study of drug efficiency or drug distribution in the tumor. For instance, a dry implant may be designed specifically for a patient and prepared in two copies: a first copy is studied *in vitro* as an avatar, to estimate if the real implantation of the second copy in the patient will be successful.

### BRIEF DESCRIPTION OF THE DRAWINGS

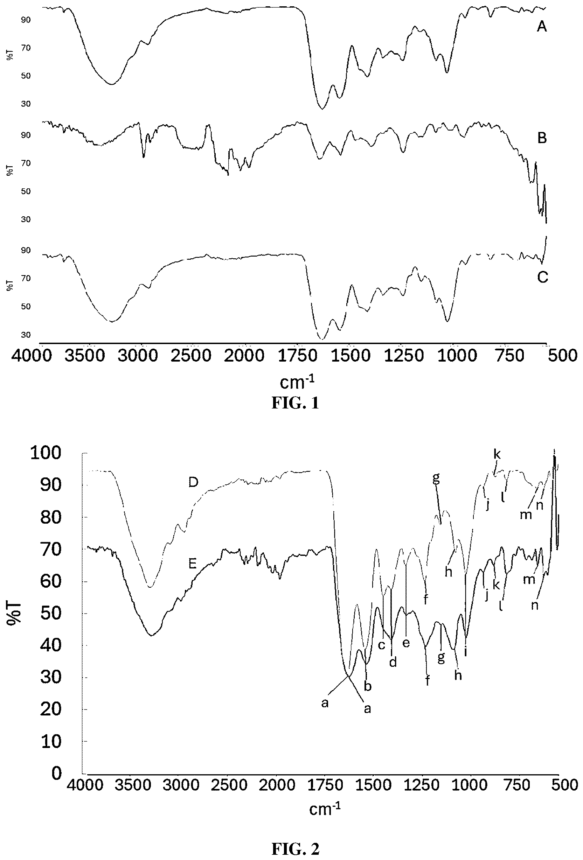

**Figure 1** is an IR spectrogram showing IR absorption (in %T - percentage of transmittance, as a function of wavenumber in cm⁻¹) for the initial implant of example 1 (Curve A), the γ-ray sterilized implant without freeze drying of example 1 (Curve B) and the freeze-dried implant of example 1 before sterilization (Curve C).
**Figure 2** is an IR spectrogram showing IR absorption (in %T - percentage of transmittance, as a function of wavenumber in cm⁻¹) for the freeze-dried implant of example 1 before sterilization (Curve D) and the γ-ray sterilized implant after freeze drying of example 1 (Curve E). Labels correspond to the following wavenumbers: a-1035.1 / b-1541 / c-1449.5 / d-1410.9 / e-1333.7 / f-1237.3 / g-1155.3 / h-1085.3 / i-1027.5 / j-935.83 / k-882.78 / 1-815.26 / m-656.1 / n-624.75.

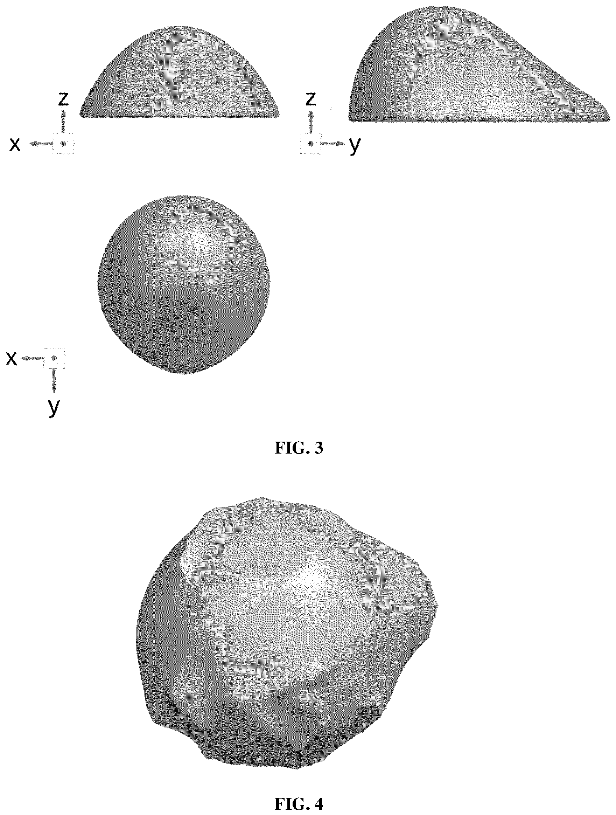

**Figure 3** shows the structure of a breast implant having a total volume of 525m³, and an isoperimetric ratio of 0.42.
**Figure** 4 shows the structure of an implant intended to replace a tumor after removal, having a total volume of 37 cm³, and an isoperimetric ratio of 0.91.

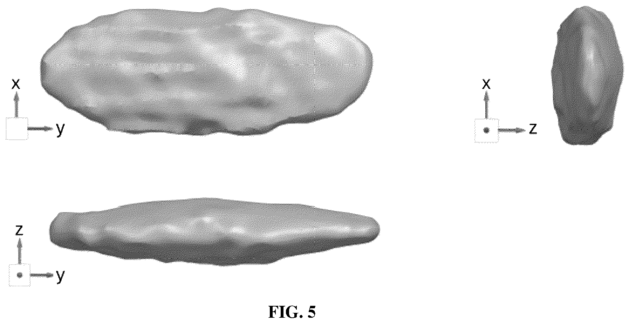

**Figure** 5 shows the structure of an implant for reconstruction surgery, having a total volume of 40 cm³, an aspect ratio of 3.4 and an isoperimetric ratio of 0.33.

### EXAMPLES

The present invention is further illustrated by the following examples.

### Measurement protocols:

### Chemical properties - IR spectrometry

Infrared measurement is carried out on a sample after drying at room temperature, leading to a dry sample suitable for FTIR analysis. This measurement makes it possible to give the chemical signature of a material and thus make it possible to observe a possible chemical modification during the drying or sterilization process. IR spectra are acquired with a PERKIN ELMER SPECTRUM 100 FTIR spectrometer in Attenuated Total Reflection (ATR) mode, with a resolution of 4 cm⁻¹. The characteristic peaks of the biocompatible structuring agent are determined as well as the characteristic peaks of the biocompatible biological agent. The impact of drying and sterilization steps can be observed by the change in characteristic peaks.

### Chemical properties - Surface energy

Surface energy of the samples is determined by contact angle measurements with three liquids (water, diiodomethane, ethylene glycol) using a goniometer - Dataphysics OCA 40 goniometer; 3 measurements for each liquid by deposition of 3 drops of 1.5 µL at 23°C - allowing to determine the dispersive component of surface energy and the polar component of surface energy. Surface energy results comprise total surface energy (mN.m⁻¹), dispersive contribution (mN.m⁻¹) and polar contribution (mN.m⁻¹), noted total/dispersive/polar in Table 1.

Surface energy is always measured with a hydrated implant - initial implant or implant freeze-dried then rehydrated.

### Mechanical properties - Traction

Test specimens are 3D printed type 2 dumbbells as defined in ISO 37 standard. The specimens have filaments of size 1 mm and pore size is 2.5 mm. Measurements are reproduced on 3 specimens, with machine elongation speed 500 mm/min, 40 mm jaw distance and test stop at breaking detection. Traction results comprise force at break (N), elongation at break (%), traction resistance (kPa) and Young modulus (kPa).

Traction test is always run with a hydrated implant - initial implant or implant freeze-dried then rehydrated. A Traction machine Criterion 45 (MTS), Sensor : 250N is used.

### Mechanical properties - Compression

Two types of specimens were used. A first type of specimens is a 3D-printed anatomical implants of size 7 cmx5 cmx2 cm and a pore size of 3 mm. Measurements are reproduced on 3 specimens, with machine speed of 20 mm/min after a pre-load of 0.02 N. Compression is made from 10 mm to 18 mm. Compression results comprise thickness variation under maximal load (%), maximal load (N) and stress at maximal load (N/mm²). A second type of specimens is a porous 3D-printed cylinder of 29 mm diameter, 13 mm thickness and filament size of 1 mm. Measurements are reproduced on 10 specimens, after a pre-load of 1 N and a compression rate of 0.1 mm/s. With porous cylinders, Young Modulus is measured (kPa).

Compression test is always run with a hydrated implant - initial implant or implant freeze-dried then rehydrated. A Traction machine Criterion 45 (MTS), Sensor: 250N is used for porous cylinder samples. An AMATEK TA1 texturometer with measurement cell LC100N is used for anatomic implants.

### Mechanical properties - Resilience

Resilience is determined by a compression test according to ISO 815 standard. A sample having the geometry of a plate is used, with H1 the initial thickness of the plate, H2 the final thickness of the plate - after compression and recovery - and H3 is the thickness of the plate under compression. Here, the samples - 3D-printed porous cylinders of 29 mm diameter and 13 mm thickness - are compressed by 25%, *i.e., H3 =* 0.75 × H1, during 24 hours at normal laboratory temperature. Resilience is finally expressed by the relation $100 \times \frac{H 1 - H 2}{H 1 - H 3}$.

The lowest the resilience, the better the material recovers its initial shape.

Resilience test is always run with a hydrated implant - initial implant or implant freeze-dried then rehydrated. A Traction machine Criterion 45 (MTS), Sensor: 250N is used for porous cylinder samples.

### Mechanical properties - Shape recovery after drying

3D printed porous cylinders of 29 mm diameter and 13 mm thickness are used here. The geometric dimensions of filaments - thickness in µm - and pores - diameter in µm - are measured by digital microscopy - Keyence VHX-700 Digital Microscope with co-axial illumination observation and pore size measurement - on the initial specimen and after drying then rehydration.

When filaments get thinner and pore size get larger, the implant is especially suitable for absorbable tissue regeneration as the overall implant porosity increases. On the contrary, filament thickening and pore size reduction after rehydration denotes a degradation of implant properties.

### Biological properties - Cell culture

3D printed hydrogel with pore size of 1000 µm and dimension of 15x15x10 mm are prepared aseptically; or freeze-dried and gamma sterilized.

Lipoaspirate of human origin is collected and used in accordance with applicable regulations. 1mL of adipose tissue was injected into the implants, corresponding to the dead volume of the implant. And then incubated at 37°C, 5% CO₂ in proliferative medium for 7 days, then in a medium adapted to the differentiation of ASCs into mature adipocytes for 18 days, with 3 renewals of the culture medium per week.

Cellular metabolic activity within the samples was studied by colorimetric analysis with Alamar Blue on culture days 4, 11, 18 and 25 after grafting. The solution was made by diluting 10 times a solution of Alamar Blue (DAL 1100, Invitrogen) in DMEM 1X (Dulbecco's Modified Eagle Medium, 31966-021, Gibco). After 3 hours of incubation at 37°C, 100 µL of the supernatants were collected in triplicate and the difference of absorbances at 570 nm and 600 nm was measured with a spectrophotometer (NanoQuant^{®} infinite M200PRO, TECAN). Differences in absorbance after 4 days and 25 days are reported in table 1.

### Biological properties - In vivo test

3D printed hydrogel implants with pore size of 1700 µm and shape of a half sphere of diameter 7 mm are prepared, either by freeze drying followed by ethylene oxide sterilization or without freeze drying - aseptically manufactured -, then implanted in rats. The flat base of the implants was placed toward the muscle while the curved top of the items was placed toward the skin. Rats were sacrificed at 4, 8, 13 and 26 weeks post-implantation, local tissue effects and degradation were assessed by macroscopic observations and histopathology.

Similar results have been obtained with 3D printed hydrogel implants either freeze dried then treated by gamma sterilization or without freeze drying.

### Example 1:

2 g of low viscosity alginate (W201502, supplied by Merck), 5 g of porcine gelatin (bloom 280 supplied by Merck) are dissolved during 12 h in 100 mL of a saline solution (NaCl, 0.1M), yielding a hydrogel.

The hydrogel is transferred into a 55 mL cartridge (Nordson EFD) equipped with an 840 µm internal diameter extrusion nozzle (Nordson EFD). The cartridge-nozzle assembly is then placed in a 3D printer (BioassemblyBot 500, Advanced Solution Lifescience, USA) allowing application of a constant pressure to the cartridge while moving in the three directions of space. The printing parameters are a speed of 25mm/sec, a pressure of 30-40PSI and a temperature of 21°C. Obtaining different filling rates is done by the internal slicer of the printer control software (Tsim, Advanced Solution Lifescience, USA), yielding an initial implant.

A stabilization solution is prepared with 4 g of transglutaminase, 4 g of CaCl₂ in 100 mL of a saline solution (NaCl, 0.1M). The stabilization solution is then brought into contact with the 3D-printed implant during 90 min at 37°C, yielding a stabilized hydrogel.

The properties of the initial implant are measured according to the protocols disclosed hereabove and presented in Table 1.

The initial implant is freeze-dried: first, the initial implant is frozen (temperature between -60°C and -20°C, normal pressure, typically 24 h), then the frozen initial implant is kept at low temperature under vacuum to induce sublimation of solid water. Sublimation of free water and water adsorbed on polymers - either polysaccharide chains or collagen or collagen derivatives molecules - is driven by the choice of temperature, pressure and duration of each step during freeze drying. Primary drying, to remove free water is typically run during several days (up to 7 days) at a pressure below 1 mbar with a temperature increasing from -55°C to 40°C. Secondary drying, to remove water adsorbed on polymers is typically run during several days (up to 7 days) at a pressure below 1 mbar with a temperature increasing from 0°C to 40°C. After freeze-drying, the dry implant is stored if necessary, at normal pressure and a temperature between 0°C and 40°C.

The dry implant is then conditioned in a bag and sterilized by gamma ray irradiation (dose 25-35 kGy - example 1a) or by exposition to ethylene oxide (180 min conditioning then 240 min exposure, followed by one day in desorption chamber - example 1b).

The sterilized dry implant is further rehydrated (immersion in a physiological saline solution for a duration between 1 min and 15 min) to yield a final implant, ready for placement. The properties of the final implant are measured and presented in Table 1 for comparison with properties of the initial implant.

### Comparative Example:

Example 1a is reproduced, except for the freeze-drying and rehydration step. The initial implant is thus sterilized by gamma ray in a wet state. The properties of the final implant are measured and presented in Table 1.

**Table 1**

| | Initial implant | Final implant Freeze drying No sterilization | Final implant 1a Freeze drying Gamma ray | Final implant 1b Freeze drying Ethylene oxide | Final implant 1a Comparative |
|---|---|---|---|---|---|
| IR spectrometry | See Fig. 1 Curve A | See Fig. 1 Curve C and D | See Fig. 2 Curve E | --- | See Fig. 1 Curve B |
| Surface energy (mN.m⁻¹) | 38.7/24.4/14.4 | 43.0/18.3/24.6 | 39.0/21.5/17.5 | 48.3/25.9/22.4 | 66.7/27.4/39.2 |
| Traction | 0.27/31.7/13.9/47.1 | 0.41/34.0/24.5/78.5 | --- | 0.45/27.3/20.1/79.5 | --- |
| Compression (anat. implant) | | 46.7/9.2/4.7 | 73.4/21.2/10.8 | 69.6/15.5/7.9 | --- |
| Compression (porous cylinder) | 373 | 330 | 322 | 374 | 236 |
| Resilience | 82 | 31 | --- | --- | 77 |
| Shape recovery (filament/pore) | 905/2422 | 777/2477 | 778/2648 | 685/2709 | 1138/2030 |
| Cell culture | Suitable 0.4/0.6 | --- | Suitable 0.3/1 | --- | --- |
| *In vivo* test | Suitable | --- | Suitable | Suitable | --- |

Comparison of IR spectrometry curves A and C show that freeze-drying does not induce a chemical modification of the hydrogel, no new bands appear or disappear on the spectrum of the lyophilized hydrogel.

When sterilization is run on hydrated implants (curve B), the peaks at 1090 cm⁻¹ and 1030 cm⁻¹ almost disappear. These peaks are representative of the elongation of the C-O group of the alginate glycosidic core. Similarly, the peak at 1410 cm⁻¹ is present to a much lesser extent than before sterilization. This could reflect a breaking in the alginate glycosidic group. In contrast, the peak at 1240 cm⁻¹ is higher after sterilization. The peak at 1540 cm⁻¹ linked to the amide groups in gelatin becomes thinner after irradiation sterilization, and the peak at 1639 cm⁻¹ is also much smaller. The broad band between 3100 cm⁻¹ and 3500 cm⁻¹ characteristic of bonded -OH bonds (hydrogen bonding, key bonds in an alginate-gelatin hydrogel) is virtually absent from the non-freeze-dried and sterilized implant's spectrum, reflecting a change in structure.

When sterilization is run on freeze-dried implants (curve E), some minor changes are observed in the characteristic spectroscopic bands, but these are less than after irradiation on hydrated implants. This comparison shows that freeze-drying allows implants to be dehydrated sufficiently for the radiolysis of water to disappear, thus avoiding the chemical reactions that might impact the implant properties.

Traction tests show that after freeze-drying and rehydration, the mechanical properties of the implants are relatively improved in terms of fracture behavior and elasticity. These mechanical properties are maintained after sterilization in the dry state and rehydration.

Compression tests on anatomical implants show that neither gamma ray nor ethylene oxide sterilization on dried implants impacts the mechanical properties of the implants. It is even observed that gamma ray sterilization improves slightly the mechanical properties of the implants.

Compression tests on porous cylinder clearly shows the decrease of Young Modulus in case of sterilization on a wet implant, whereas sterilization on a freeze-dried implant leads to a conservation of the Young Modulus.

Resilience tests show that freeze-drying brings a clear improvement in mechanical properties. Indeed, the residual deformation of initial implants is of the order of 80% and the residual deformation of comparative implants is of the order of 77%, while the residual deformation of implants after freeze-drying and rehydration is of the order of 30%. This property is very advantageous in the case of body implants.

Shape recovery tests show that samples sterilized without freeze drying show a slight pore retraction and filament thickening. On the contrary, freeze dried samples show a higher porosity and thinner filaments, and sterilization has almost no incidence on the geometry of implant: this is an advantage in porous implant design for absorbable tissue regeneration.

Regarding cell culture: after 25 days of culture, the Alamar blue assay shows that adipose tissue metabolic activity increases more over time in freeze-dried implants than in standard implants.

Regarding *in vivo* tests: all animals were clinically normal at baseline and throughout the study. Macroscopically, no abnormalities were observed in the subcutaneous tissue in contact with the implants, regardless of the timepoint. 4, 8, and 13 weeks after implantation, all implants were visible and had a similar color and shape to the original surgery. 26 weeks after implantation, the diameter of the implants appeared to be slightly reduced compared to the implantation (5 to 6 mm radius) and some devices had irregular edges or were slightly flattened. These changes in diameter and appearance reflect the ongoing degradation process.

Microscopically, local tissue effects were consistent with a normal foreign body response that decreased in intensity over time: moderate to marked at 4 weeks, mild to moderate at 8 weeks and 13 weeks, and mild at 26 weeks. In the implanted sites, a mild to moderate amount of newly formed fibrovascular connective tissue surrounded and infiltrated the material of the articles tested. This connective tissue became more mature over time and its extent tended to increase from week 13 onwards. The capsule delineating the implantation cavity was thin and did not prevent the interaction of the biomaterial with the host tissue. The degradation of the polymers constituting the implant was considered mild to moderate at all timepoints. Microscopic evidence of phagocytosis of the material was observed at all time points, it was greater at the 4-week time point and then decreased over time. After 26 weeks, test article degradation was estimated at about 40-50%.

No significant differences in tolerance or degradation were observed *in vivo* in rodents between freeze-dried and sterilized implants as compared to non-lyophilized implants. This indicates that the freeze-drying process for the manufacture of regenerative implants is suitable.

Results of cell culture and *in-vivo* tests are summarized globally as histology in table 2. A good histology means that tissue inside the implant is healthy and functional, with sane cells and extracellular matrix.

### Examples 2-4:

Example 1 is reproduced, but parameters are changed as shown in table 2.

**Table 2**

| Example | biocompatible Structuring agent | Biocompatible biological agent | Initial implant structure (incl. q1/aspect ratio) | Drying conditions | Sterilization conditions | Properties recovery | Histology |
|---|---|---|---|---|---|---|---|
| 1a | Alginate | Porcine gelatin | 15x15x10mm q1=0.5 pore:1000µm | Freezing Primary drying Secondary drying | Gamma ray (25-35 kGy) | See Table 1 | Good |
| 1b | Alginate | Porcine gelatin | 15x15x10mm q1=0.5 pore: 1000µm | Freezing Primary drying Secondary drying | ethylene oxide 240 min | See Table 1 | Good |
| C.ex 1a | Alginate | Porcine gelatin | 15x15x10mm q1=0.5 pore:1000µm | None | Gamma ray (25-35 kGy) | See Table 1 | Good |
| 2 | Alginate | Porcine gelatin | See Fig. 3 q1=0.42 | Freezing Primary drying Secondary drying | Gamma ray (25-35 kGy) | Like 1a | Good |
| 3 | Alginate | Porcine gelatin | See Fig. 4 q1=0.91 | Freezing Primary drying Secondary drying | Gamma ray (25-35 kGy) | Like 1a | --- |
| 4 | Alginate | Porcine gelatin | See Fig. 5 q1=0.33 | Freezing Primary drying Secondary drying | Gamma ray (25-35 kGy) | Like 1a | --- |

## Claims

1. A dry implant comprising a dried hydrogel, wherein said dried hydrogel comprises i) at least one biocompatible structuring agent, wherein said at least one biocompatible structuring agent is a polysaccharide network, and ii) at least one biocompatible biological agent, wherein said at least one biocompatible biological agent is a native collagen network or a network of a derivative of collagen, wherein said dry implant comprises at least one porous zone, wherein the at least one porous zone comprises a plurality of pores, the at least one porous zone having a void fraction ranging from about 5% to about 99.5%.

2. The dry implant according to claim **1** wherein the implant has an isoperimetric ratio greater than or equal to 0.3, or an aspect ratio greater than or equal to 3.

3. The dry implant according to any one of claims **1** to **2,** wherein the at least one biocompatible structuring agent is selected from the group consisting of alginate, hyaluronic acid, cellulose and its derivatives, chitosan and its derivatives, dextran, heparan sulfate, agarose, chondroitin and chondroitin sulfate, glycogen, starch, carrageenan, pectin, xanthan gum, pullulan, gellan, guar guar, gum arabic, and combinations thereof; preferably alginate, hyaluronic acid, cellulose and its derivatives, and combinations thereof; more preferably alginate.

4. The dry implant according to any one of claims **1** to **3,** wherein the at least one biocompatible biological agent is selected from the group consisting of native collagen, gelatin, or chemically modified collagens; preferably said at least one biocompatible biological agent is gelatin.

5. The dry implant according to any one of claims **1** to **4,** wherein the at least one biocompatible structuring agent is alginate and wherein the at least one biocompatible biological agent is gelatin.

6. The dry implant according to any one of claims **1** to **5,** wherein the at least one porous zone occupies 90% or more of the volume of the dry implant.

7. The dry implant according to any one of claims **1** to 6, wherein the dry implant comprises at least one non-porous zone, wherein said at least non-porous zone is partially surrounding the porous zone.

8. The dry implant according to any one of claims **1** to **7,** wherein the dry implant is sterile.

9. A process for manufacturing a dry implant comprising successively:
a) a step of preparing a hydrogel, wherein said hydrogel comprises i) at least one biocompatible structuring agent, wherein said at least one biocompatible structuring agent is a polysaccharide, and ii) at least one biocompatible biological agent, wherein said at least one biocompatible biological agent is a native collagen or a derivative thereof,
b) a step of three-dimensionally shaping the hydrogel so as to form a three-dimensional body implant comprising at least one porous zone, wherein the at least one porous zone comprises a plurality of pores, and wherein the at least one porous zone having a percentage of void ranging from about 5% to about 95%,
c) a step of stabilizing the hydrogel,
d) a step of drying, preferably freeze-drying, the stabilized hydrogel to obtain a dry implant.

10. The process according to claim **9,** further comprising a step e) of sterilizing the dry implant, preferably wherein the sterilization is done by one or more of the following methods: sterilization by irradiation, sterilization by plasma, sterilization by gas such as ethylene oxide, or sterilization by supercritical carbon dioxide.

11. The process according to anyone of claims **9 to 10,** wherein the at least one biocompatible structuring agent is selected from the group consisting of alginate, hyaluronic acid, cellulose and its derivatives, chitosan and its derivatives, dextran, heparan sulfate, agarose, chondroitin and chondroitin sulfate, glycogen, starch, carrageenan, pectin, xanthan gum, pullulan, gellan, guar guar, gum arabic, and combinations thereof, preferably wherein the at least one biocompatible structuring agent is alginate.

12. The process according to any one of claims **9** to **11,** wherein the at least one biocompatible biological agent is selected from the **group** consisting of native collagen, gelatin, chemically modified collagens; preferably said at least one biocompatible biological agent is gelatin.

13. The process according to any one of claims **9 to 12,** wherein the at least one biocompatible structuring agent is alginate and wherein the at least one biocompatible biological agent is gelatin and wherein, in step c), alginate is cross-linked with at least one divalent cation, preferably calcium, and gelatine is cross-linked with transglutaminase.

14. The process according to any one of claims **9** to **13,** wherein the dry implant has an isoperimetric ratio greater than or equal to 0.3, or an aspect ratio greater than or equal to 3.

15. A three-dimensional implant obtainable by a process for manufacturing a dry implant according to any one of claims **9** to **14.**
